(19) Europäisches Patentamt
European Patent Office
Office européen des brevets

(11) EP 4 273 254 A1

(12) EUROPEAN PATENT APPLICATION

(43) Date of publication:
**08.11.2023 Bulletin 2023/45**

(21) Application number: **22172031.1**

(22) Date of filing: **06.05.2022**

(51) International Patent Classification (IPC):
***C12P 7/40*** *(2006.01)*

(52) Cooperative Patent Classification (CPC):
**C12P 7/40; C12P 13/04; C12Y 206/01**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Enzymicals AG**
  **17489 Greifswald (DE)**
- **Universität Greifswald**
  **17487 Greifswald (DE)**

(72) Inventors:
- **Höhne, Matthias**
  **17487 Greifswald (DE)**
- **Kollipara, Manideep**
  **17487 Greifswald (DE)**
- **Tumkur Srinivasamurthy, Vishnu Sinha**
  **17489 Greifswald (DE)**

(74) Representative: **Altmann Stößel Dick Patentanwälte PartG mbB**
**Theodor-Heuss-Anlage 2**
**68165 Mannheim (DE)**

Remarks:
The complete document including Reference Table(s) and the Sequence Listing(s) can be downloaded from the EPO website

(54) **ENZYMATIC METHOD FOR PREPARING (R)-3-AMINO-4-ARYL-BUTANOIC ACID DERIVATIVES**

(57) In a first aspect, the invention relates to a method for preparing a compound of formula (II)

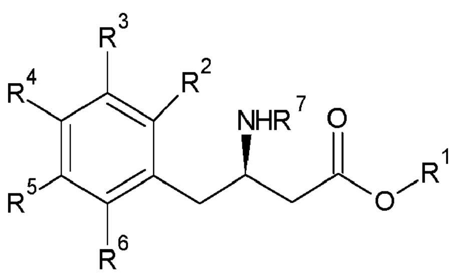

(II)

wherein
$R^1$
is selected from the group consisting of hydrogen atom, C1 to C10 alkyl, C6 to C12 aryl, C1 to C5 alkyl-C6 to C12 aryl, C3 to C10 heteroaryl, and C1 to C5
$R^2$, $R^3$, $R^4$,
alkyl-C3 to C10 heteroaryl;
$R^5$, $R^6$
are independently of each other either a hydrogen atom or a halogen atom;
$R^7$
is a hydrogen atom or a protective group.

A second aspect of the invention is directed to a compound of formula (II) obtained or obtainable from the method of the first aspect. In a third aspect, the invention is related to the use of the compound of formula (II) obtained or obtainable from the method of the first aspect or the compound of formula (II) according to the second aspect for the preparation of sitagliptin, retagliptin or evogliptin. A fourth aspect of the invention relates to a process for preparing sitagliptin or retagliptin or evogliptin based on the compound of formula (II) obtained or obtainable from the method of the first aspect or the compound of formula (II) according to the second aspect.

EP 4 273 254 A1

## Description

In a first aspect, the invention relates to a method for preparing a compound of formula (II)

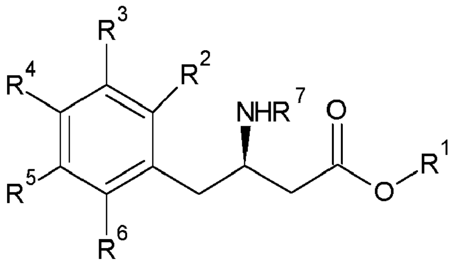

(II)

wherein

| | |
|---|---|
| $R^1$ | is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl; |
| $R^2$, $R^3$, $R^4$, $R^5$, $R^6$ | are independently of each other either a hydrogen atom or a halogen atom; |
| $R^7$ | is a hydrogen atom or a protective group. |

A second aspect of the invention is directed to a compound of formula (II) obtained or obtainable from the method of the first aspect. In a third aspect, the invention is related to the use of the compound of formula (II) obtained or obtainable from the method of the first aspect or the compound of formula (II) according to the second aspect for the preparation of sitagliptin, retagliptin or evogliptin. A fourth aspect of the invention relates to a process for preparing sitagliptin or retagliptin or evogliptin based on the compound of formula (II) obtained or obtainable from the method of the first aspect or based on the compound of formula (II) according to the second aspect.

(*R*)-3-amino-4-aryl-butanoic acid derivatives are important for several drugs. For example, the (*R*)-3-amino-4-aryl-butanoic acid derivative sitagliptin is an oral anti-diabetic drug and acts as dipeptidyl peptidase-4 (DPP-4) inhibitor. In addition to its chemical synthesis, an enzymatic preparation has been developed for sitagliptin that employs an (*R*)-selective amine transaminase (ATA) ATA-117-rd11, which was generated by extensive protein engineering (Scheme 1) (Savile et al., Science 2010, 329, 305).

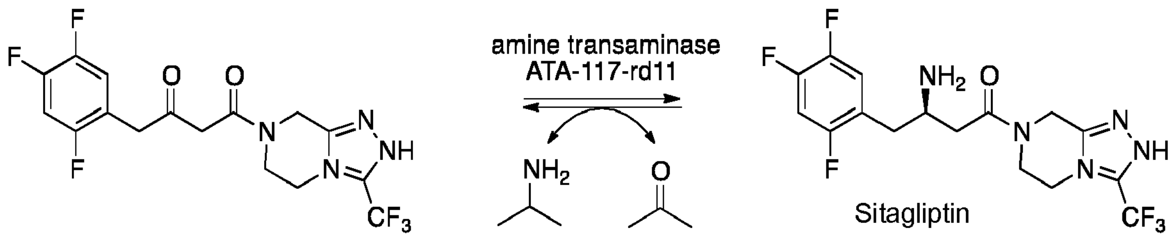


Scheme 1: Synthesis of a sitagliptin via transamination

An alternative synthesis approach via transamination is the preparation of an intermediate, (*R*)-3-amino-4-(2,4,5-trifluorophenyl)butanoate alkyl ester 2, which later can be converted into sitagliptin or other compounds by subsequent chemical transformations (Scheme 2).

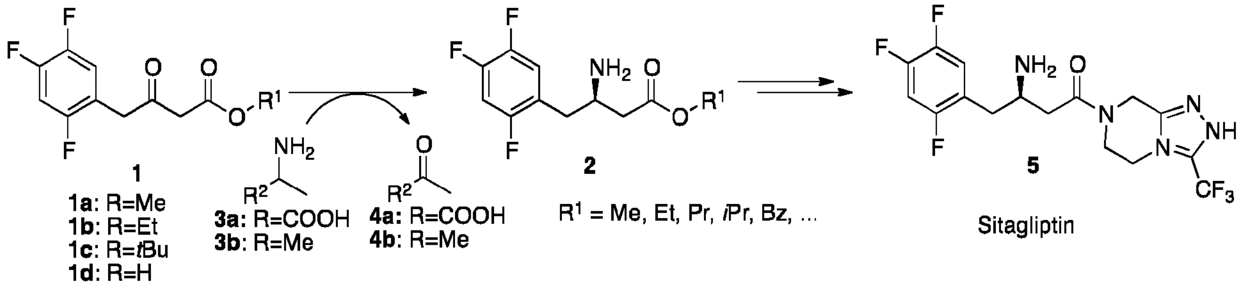

1

**1a**: R=Me
**1b**: R=Et
**1c**: R=*t*Bu
**1d**: R=H

**3a:** R=COOH
**3b**: R=Me

**4a:** R=COOH
**4b**: R=Me

2

$R^1$ = Me, Et, Pr, *i*Pr, Bz, ...

5

Sitagliptin

Scheme 2: Synthesis of a sitagliptin precursor molecule 2 via transamination.

Several patents/patent applications related to this route are published and a variety of suitable enzymes has been identified so far. Indian patent IN 1391CHE2014 A discloses the feasibility of this approach using 1a and various other alkyl ester derivatives. As enzymes, the large ATA collection from Codexis was screened. However, enantiomeric purities obtained in the product were not perfect (<93 % ee). Subsequent patents/patent applications provided improved enzymes (wild types and variants) to increase the enantiomeric purity or to provide enzyme formulations that facilitate reusability. CN 104805069 B discloses an immobilized ATA preparation using the enzyme from *Mycobacterium vanbalenii* entrapped in alginate beads, which was reusable for >20 cycles. CN 106801043 A reports on an engineered variant of *Aspergillus terreus* transaminase and a scale-up to the conversion of 32 kg of ketone substrate (in a 2000 liter reactor). CN 106191148 A includes a commercial transaminase from unknown source and the use of the *tert* butyl ester 1c as a substrate instead of the methyl or ethyl esters, which were employed in the previous patents. This results in a superior stability of the ester substrate and product.

WO 2019/128894 A1 also discloses engineered transaminase variants. It is argued that wild type transaminases are usually not stable enough or do not have the desired substrate specificity and thus this application discloses results from an extensive protein engineering to provide improved variants. It is exemplified with the model substrate acetophenone to yield 1-phenylethylamine, and the substrate ketone 1 is only one substrate of a large collection. WO 2019/007146 A1 also reports improved ATA variants that were generated by protein engineering.

A scientific publication (Hou et al, Tetrahedron 2016, 72, 4660) examined the activity of the enzyme ATA-117-rd11 (the transaminase originally evolved for making sitagliptin) towards various substrate ketones and found the best ester in terms of solubility is **1** with $R^1$ = $-CH_2CH_2OH$. It can be used in higher concentrations compared to the other esters what results in higher enzyme activities.

Compared to the ester substrates, also the free acid **1d** can be employed as a substrate. This is described in CN 105331651 A. As enzyme, a D-amino acid dehydrogenase is used for aminating the keto acid with $NH_3$ and NADH to form amino acid **2d.** Another approach is described in Kim et al, Enzyme Microb. Technol. 2019, 120, 52. Here, the free keto acid **1d** is obtained from the ethyl ester **1b** by the action of a lipase and subsequently aminated by a transaminase from *Ilumatobacter coccineus.* The difficulty when using the keto acid is its instability, as it is prone to decarboxylation and thus a precise adjustment of lipase and transaminase activities in the reaction is required in order to prevent the accumulation of the keto acid 1d.

The following challenges are associated with manufacturing the sitagliptin intermediate 2:

1) Substrate and product stability: The substrates and products are instable and prone to hydrolysis (Scheme 3).

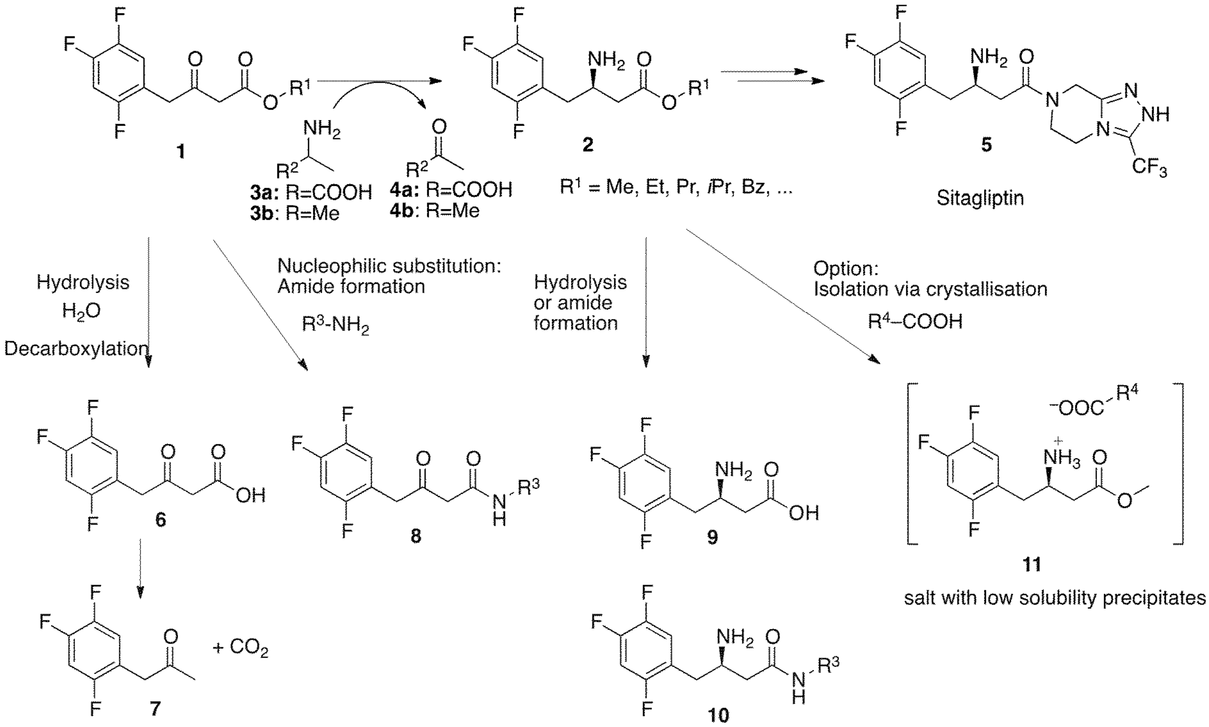


**Scheme 3: Possible side reactions of substrates and products**

In general, ketones and amines are quite stable in the reaction solution despite the increased pH and temperature used in the transamination reaction. However, isopropylamine had to be avoided in some processes as the ketone substrate or amine product can be sensitive to high isopropylamine concentrations, leading to side product formation, diminished product yields and more complicated work-up procedures (Busto et al., Adv. Synth. Catal. 2014, 356, 1937). As an alternative amino donor, alanine was used in this study as it turned out to be less aggressive. A similar situation is observed in this investigated conversion of substrate **1** to form product **2.**

The generation of hydrolysis product **9** under enzymatic reaction conditions is reported (Hou et al, Tetrahedron 2016, 72, 4660) for various esters an amount of up to 30 % of the conversion was the hydrolysis product **9.** This amino acid is difficult to isolate because it cannot be extracted; introduction of Boc-protection solves this problem but complicates the process by addition of several process steps.

The instability can also be solved by using the *tert*-butyl ester of **1** ($R^1$=*t*-Bu), but *tert*-butylesters are more difficult and expensive in the manufacturing. Other (bulky) esters that lead to more stable products are not well accepted by the investigated enzyme ATA-117 and will also decrease atom economy.

Additionally, hydrolysis of the substrate is possible under reaction conditions, and also the nucleophilic substitution of the esters **1** and **2** with isopropylamine leading to amides **8** and **10** as byproducts is possible.

2) Product inhibition of the transaminase and equilibrium: Often, transaminase reactions suffer from unfavorable equilibrium and product inhibitions. Usually, the co-product acetone has to be removed to drive the equilibrium. The product inhibition by the produced amine product can often only be circumvented by protein engineering.

The stability of sensitive substrates and products was shown to be increased if alanine is used as amino donor instead of isopropylamine (Busto et al., Adv. Synth. Catal. 2014, 356, 1937). All transaminases identified so far for the synthesis of **2** have been identified in the family of the (*R*)-selective transaminases. However, many of the previously employed transaminases are engineered to accept isopropylamine and thus are not able to efficiently use alanine as amino donor. Furthermore, all efficient literature-known amine transaminases with (*R*)-selectivity only accept D-alanine but are resistant towards L-alanine. As D-alanine is much more expensive compared to L-alanine, it would be highly desirable to identify a transaminase that prefers L-alanine or another available L-amino acid as amino donor but on the same time produces the desired (*R*)-amino ester **2.** Even IN 1391CHE2014 A, which mentions L-alanine within a large group of possible amine donors, uses only isopropylamine as amine donor in the experimental section and thus it is not known how efficient L-amino acids are accepted by the enzyme.

Thus, the object was the provision of a transaminase, which enables stereopreference for the (*R*)-amino ester **2** and a high stereoselectivity.

The problem was solved according to a first aspect of the invention by a method for preparing a compound of formula (II)

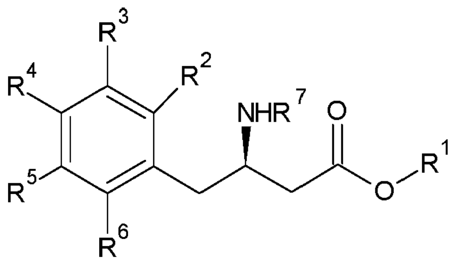

(II)

wherein

| | |
|---|---|
| $R^1$ | is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl; |
| $R^2$, $R^3$, $R^4$, $R^5$, $R^6$ | are independently of each other either a hydrogen atom or a halogen atom; |
| $R^7$ | is a hydrogen atom or a protective group; |

the method comprising:

(a) Providing a compound of formula (I)

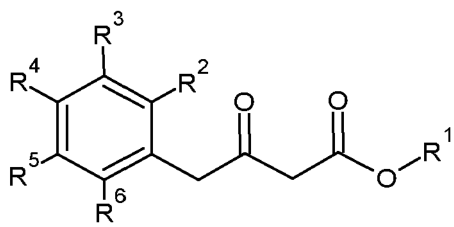

(I)

wherein $R^1$ is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl;
$R^2$, $R^3$, $R^4$, $R^5$ and $R^6$ have the same meaning as in the compound of formula (II);
(b) Providing an amine donor comprising an $-NH_2$ group;
(c) Reacting the compound of formula (I) of (a) with the amine donor of (b) in the presence of an amine transaminase, wherein the amine transaminase comprises a polypeptide having at least 80 % sequence identity with the polypeptide of SEQ ID NO. 1; thereby obtaining a reaction mixture comprising a compound of formula (II).

Preferably, $R^1$ in formula (I) is the same or different as $R^1$ in formula (II).

In some embodiments of the method for preparing a compound of formula (II), the compound of formula (II) is a pure compound having only one $R^1$ group, wherein $R^1$ is selected from the group indicated above. In some embodiments, the compound of formula (II) comprises at least two compounds (IIa) and (IIb) wherein $R^1$ is a hydrogen atom in compound (IIa) and $R^1$ is selected from the group consisting of C1 to C10 alkyl, C6 to C12 aryl, C1 to C5 alkyl-C6 to C12 aryl, C3 to C10 heteroaryl, and C1 to C5 alkyl-C3 to C10 heteroaryl in compound (IIb).

It was surprisingly found that using an amine transaminase comprising a polypeptide having at least 80 % sequence identity with the polypeptide of SEQ ID NO. 1 for reacting the compound of formula (I) of (a) with the amine donor of (b) resulted in an enantio-preference for the production of (*R*)-ester and/or (*R*)-acid.

The transaminase having an amino acid sequence according to SEQ ID NO. 1 is listed in the UniProt database as E1V8W4 (E1V8W4_HALED). The underlying DNA sequence has Genebank accession number MT828898. The finding that said transaminase transforms a compound of formula (I) of (a) with the amine donor of (b) into a compound of formula (II) with enantio-preference for the production of (*R*)-ester and/or (*R*)-acids is somewhat surprising for at least two reasons: First, the transaminase from *Halomonas elongata* is a wild type transaminase and was not expected to accept such a bulky substrate as a compound of formula (I). Secondly, the enzyme showed almost no activity for most of the typical substrates such as 1-phenylethylamine and thus seemed to be a very inefficient amine transaminase - The discovery and characterization of the transaminase from *Halomonas elongata* (amongst others) is described in the manuscript "Fingerprint-guided characterization of proteins from the 3N5M superfamily reveals an amine transaminase with high thermal and operational stability", which is in the revision process and not yet published.

In some embodiments of the method for preparing a compound of formula (II), the amine transaminase of (c)

comprises a polypeptide having at least 80 % sequence identity, preferably at least 85 % sequence identity, more preferably at least 90 % sequence identity, more preferably at least 95 % sequence identity, more preferably at least 98 % sequence identity, more preferably at least 99 % sequence identity, more preferably at least 99.5 % sequence identity, with the polypeptide of SEQ ID NO. 1, wherein more preferably the amine transaminase of (c) consists of a polypeptide having at least 85 % sequence identity, preferably at least 90 % sequence identity, more preferably at least 95 % sequence identity, more preferably at least 98 % sequence identity, more preferably at least 99 % sequence identity, more preferably at least 99.5 % sequence identity, with the polypeptide of SEQ ID NO. 1. An identity of xx % means that sequences are comprised, which have an amino acid sequence which differs due to at least one substitution, deletion, insertion and/or addition from the indicated sequence of SEQ ID NO. 1. An added and/or exchanged amino acid residue can either be codable amino acid residues or other naturally occurring residues or purely synthetic amino acid residues. The term "at least one", as used herein means one, or more than one, such as "at least two", "at least three", "at least four", "at least five", etc.. The degree of identity (e.g. expressed as "% sequence identity") between two amino acid sequences can be determined by algorithms well known in the art. Preferably, the degree of identity is determined by comparing two optimally aligned sequences over a comparison window, where the fragment of sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP, BESTFIT or BLAST (Basic Local Alignment Search Tool) is preferably employed to determine their optimal alignment and, thus, the degree of identity.

Irrespective whether the compound of formula (II) is a pure compound or a mixture of at least (IIa) and (lib), at least 90% of the compound of formula (II) or of the compounds of formula (IIa) and (IIb) have (*R*)-configuration, preferably at least 95% of the compound of formula (II) or the compounds of formula (IIa) and (IIb) have (*R*)-configuration, more preferably at least 96% of the compound of formula (II) or the compounds of formula (IIa) and (IIb) respectively have (*R*)-configuration, more preferably at least 97% of the compound of formula (II) or the compounds of formula (IIa) and (IIb) respectively have (*R*)-configuration, more preferably at least 98% of the compound of formula (II) or of the compounds of formula (IIa) and (IIb) respectively have (*R*)-configuration, each with respect to the C-atom bearing the $NHR^7$ group in formula (II).

The amine donor comprising an $-NH_2$ group is preferably selected from the group consisting of ethylamine, isopropylamine, butylamine, L-alanine, , L-phenylalanine, , L-glutamine, L-leucine, 3-aminobutyric acid, 1-phenylethanamine, (S)-phenylethanamine, 2-amino-4-phenylbutane, glycine, L-glutamic acid, , L-aspartic acid, L-lysine, L-ornithine, beta-alanine, taurine, n-octylamine, cyclohexylamine, 1,4-butanediamine, 1,6-hexanediamine, 6-amino hexanoic acid, 4-aminobutyric acid, tyramine, benzyl amine, 2-aminobutane, 2- amino-1-butanol, 1-amino-1-phenylethane, 1-amino-1-(2-methoxy-5-fluorophenyl) ethane, 1-amino-1-phenylpropane, 1-amino-L-(4-hydroxyphenyl) propane, 1-amino-1-(4-bromophenyl)propane, 1-amino-1-(4-nitrophenyl)propane, 1-phenyl-2-amino propane, L-(3-trifluoromethylphenyl)-2-aminopropane, 2-aminopropanol, 1-amino-1-phenylbutane, 1-phenyl-2-aminobutane, 1-(2,5-dimethoxy-4-methylphenyl)-2-amino butane, L-phenyl-3-aminobutane, L-(4-hydroxyphenyl)-3-aminobutane, L-amino-2-methylcyclopentane, L-amino-3-methylcyclopentane, L-amino-2-methylcyclohexane, 1-amino-1-(2-naphthyl)ethane, 3-methylcyclopentylamine, 2-methylcyclo pentylamine, 2-ethylcyclopentylamine, 2-methylcyclohexylamine, 3-methylcyclohexylamine, 1-aminotetralin, 2-aminotetralin, 2-amino-5-methoxytetralin, and 1-aminoindane, L-threonine, L-cysteine, tryptophan, serine, phenylglycinol, tyrosine, glutamine, methionine, 5-amino valeric, 8-amino caproic acid, 2-aminobutaric acid, D-2-galactosamine, ethyl 3-aminobutyrate, 3-amino-3-phenylpropionic acid, mixtures of two or more of these amine donors, the racemates of these amine donors, salts of these amine donors or of their racemates, and mixtures of one or more of these amine donors with one or more salt(s) of these amine donors, wherein the amine donor comprising an $-NH_2$ group is preferably selected from the group consisting of isopropylamine, L-alanine, , phenylalanine, glutamine, 3-aminobutyric acid, 2-amino-4-phenylbutane, L-glutamic acid, L-aspartic acid, beta-alanine, 6-amino hexanoic acid, 5-amino valeric acid, 8-amino caproic acid, 4-aminobutyric acid, 2-aminobutaric acid, 1-phenyl-2-amino propane, L-(3-trifluoromethylphenyl)-2-aminopropane, L-phenyl-3-aminobutane, L-(4-hydroxyphenyl)-3-aminobutane, D-2-galactosamine, ethyl 3-aminobutyrate, 3-amino-3-phenylpropionic acid, mixtures of two or more of these amine donors, salts of these amine donors, and mixtures of one or more of these amine donors with one or more salt(s) of these amine donors, wherein the amine donor comprising an $-NH_2$ group is more preferably selected from L-alanine, L-aspartic acid mixtures of two or more of these amine donors, the racemates of these amine donors, salts of these amine donors or of their racemates, and mixtures of one or more of these amine donors with one or more salt(s) of these amine donors and/or

with one or more of their racemates, more preferably the amine donor comprising an $-NH_2$ group is selected from L-alanine, racemate comprising L-alanine, L-aspartic acid, racemate comprising L-aspartic acid, a mixture of L-alanine and L-aspartic acid, mixture of racemate comprising L-alanine and L-aspatic acid, mixture of L-alanine with racemate comprising L-aspartic acid, salts of L-alanine, salts of racemate comprising L-aspartic acid, salts of L-aspartic acid, salts of racemate comprising L-aspartic acid, mixtures of L-alanine with one or more salt(s) of L-aspartic acid, mixtures of racemate comprising L-alanine with one or more salt(s) of L-aspartic acid, mixtures of one or more salt(s) of L-alanine with L-aspartic acid and mixtures of one or more salt(s) of L-alanine with racemate comprising L-aspartic acid.

In some embodiments, the amine donor comprising an $-NH_2$ group is preferably selected from the group consisting of L-alanine, L-aspartic acid, salts of L-aspartic acid, salts of L-alanine, and mixtures of two or more of these amine donors.

In embodiments of the method for preparing a compound of formula (II) where alanine is employed as amine donor comprising an $-NH_2$ group, it is preferred to drive the reaction forward by pyruvate removal. Methods for pyruvate removal are known to the skilled person and preferably comprise one or more additional enzyme(s) for removing the formed co-product to drive the reaction equilibrium,
e.g. if pyruvate is the co-product, pyruvate removal is preferably done by one or more techniques selected from (i) to (iii):

(i) decarboxylation with pyruvate decarboxylase to acetaldehyde,
(ii) reduction to lactic acid employing lactate dehydrogenase and
(iii) reductive amination to alanine employing alanine dehydrogenase.

Especially for (ii), (iii), a cofactor regeneration is preferably performed by further enzymes such as glucose dehydrogenase or formate dehydrogenase.

In some embodiments of the method for preparing a compound of formula (II), $R^3$, $R^4$ and $R^6$ in the compound of formula (I) and in the compound of formula (II) are each a halogen atom, independently selected from the group consisting of fluorine atom, bromine atom, chlorine atom and iodine atom, preferably $R^3$, $R^4$ and $R^6$ are each the same halogen atom selected from the group consisting of fluorine atom, bromine atom, chlorine atom and iodine atom, more preferably, $R^3$, $R^4$ and $R^6$ are each a fluorine atom; and $R^2$ and $R^5$ are each a hydrogen atom.

In some embodiments of the method for preparing a compound of formula (II), $R^1$ in in the compound of formula (I) is a hydrogen atom or a C1 to C10 alkyl group, preferably a hydrogen atom or a C1 to C5 alkyl group.

In some embodiments of the method for preparing a compound of formula (II), $R^7$ in the compound of formula (II) is a hydrogen atom or a protective group selected from the group consisting of *tert*-butyloxy carbonyl (BOC), benzyloxycarbonyl (Cbz), fluorenylmethylenoxycarbonyl (Fmoc) and allyloxycarbonyl (Alloc), wherein $R^7$ is preferably a hydrogen atom or a Boc group.

In some embodiments of the method for preparing a compound of formula (II), reacting in (c) is done at a temperature in the range of from 10 to 55 °C, preferably in the range of from 15 to 45°C, more preferably in the range of from 20 to 40 °C, more preferably in the range of from 25 to 30°C.

In some embodiments of the method for preparing a compound of formula (II), reacting in (c) is done at a pressure in the range of from 20 mbar to 2 bar, preferably in the range of from 100 mbar to 1.5 bar, more preferably in the range of from 800 to 1300 mbar, more preferably in the range of from 900 to 1100 mbar.

In some embodiments of the method for preparing a compound of formula (II), reacting in (c) is done in solution, preferably in aqueous solution, wherein the aqueous solution preferably comprises at least 60 weight-%, more preferably at least 80 weight-%, more preferably at least 95 weight-%, more preferably at least 98 weight-%, water, based on the overall weight of the aqueous solution.

In some embodiments of the method for preparing a compound of formula (II), the aqueous solution comprises an organic solvent, wherein the aqueous solution preferably comprises at the outmost 40 weight-%, more preferably at the outmost 20 weight-%, more preferably at the outmost 5 weight-%, more preferably a the outmost 2 weight-% organic solvent, based on the overall weight of the aqueous solution, wherein the organic solvent is preferably a polar organic solvent having an n-octanol/Water partition coefficient (Kow) in the range of from 1 to 25, preferably in the range of from 2 to 22, at 298 K, preferably the organic solvent is selected from the group consisting of dimethyl sulfoxide (DMSO, Kow = 2), methanol (Kow = 19), ethanol (Kow = 22) 2,2,2- trifluoroethanol (Kow = 2), N,N-Dimethylformamide (DMF, Kow = 4), ethylene glycol (1,2-ethandiol, Kow = 4), propylene glycol (1,2-propane diol, Kow = 4), and mixtures of two or more of these solvents. The weight-% of water and organic solvent are each based on the pure aqueous solution, prior to the addition of educts, products etc. The KOW values indicated are derived from the Dortmunder Datenbank, DDB, http://ddbonline.ddbst.com/DDBSearch.

In some embodiments of the method for preparing a compound of formula (II), reacting in (c) is done in buffered solution, wherein the buffer is preferably selected from the group of (4-(2-hydroxyethyl)-1-piperazineethanesulfonic acid) buffer (HEPES), N-cyclohexyl-2-aminoethane sulfonic acid buffer (CHES), sodium phosphate buffer (Napp), potassium phosphate buffer (Kapp), tris(hydroxymethyl)aminomethane hydrochloride buffer (Tris-HCl), buffer comprising a mixture

of tris base, acetic acid and EDTA (TAE), 3-(N-morpholino)propanesulfonic acid buffer (MOPS), borate buffered saline (BBS), phosphate-buffered saline (PBS) and mixtures of two or more of these buffers, more preferably a buffer selected from the group of Napp, HEPES, CHES, CAPS and mixtures of two or more of these buffers, more preferably a buffer selected from the group of Napp, HEPES, CHES and mixtures of two or more of these buffers, more preferably a buffer selected from the group of HEPES, CHES and mixtures of these buffers, more preferably the buffer comprises at least CHES.

In some embodiments of the method for preparing a compound of formula (II), reacting in (c) is done at a pH value in the range of from 6 to 11, preferably in the range of from 8 to 11, more preferably in the range of from 9 to 10.5, more preferably in the range of from 9.75 to 10.25.

In some embodiments of the method for preparing a compound of formula (II), reacting in (c) is done at a concentration of the compound of formula (I) in the range of from 0.1 to 1000 mM, preferably in the range of from 1 to 200 mM, more preferably in the range of from 3 to 90 mM, more preferably in the range of from 5 to 80 mM.

In some embodiments of the method for preparing a compound of formula (II), reacting in (c) is done with a ratio of the amine donor of (b) to the compound of formula (I) in the range of from 1:1 to 100:1, preferably in the range of from 1:1 to 50:1, more preferably 1:1 to 30:1, more preferably in the range of from 1:1 to 25:1.

In some embodiments of the method for preparing a compound of formula (II), reacting in (c) is done in the presence of a cofactor, preferably pyridoxal 5'-phosphate (PLP), wherein the cofactor, preferably PLP, preferably has a concentration in the range of from 0.01-5 mM.

In some embodiments of the method for preparing a compound of formula (II), reacting in (c) is done in the presence of a further enzyme, preferably selected from the group consisting of lactate dehydrogenase, glucose dehydrogenase, alanine dehydrogenase, formate dehydrogenase and a mixture of at least two of these further enzymes (equilibrium shift by enzyme cascades).

In some embodiments of the method for preparing a compound of formula (II), reacting in (c) is done in the presence of a carboxylic acid of formula (III)

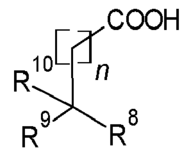

(III)

wherein:

n is zero or 1;

the residues $R^8$ and $R^9$ together form a fluorine ring or are both phenyl or together form a phenyl ring, wherein each phenyl ring has at least one further substituent selected from the group consisting of hydrogen atom, halogen atom, and nitro group; and the residue $R^{10}$ is hydrogen atom or methyl or absent if $R^8$ and $R^9$ together form a phenyl ring; thereby obtaining a salt of formula (IV)

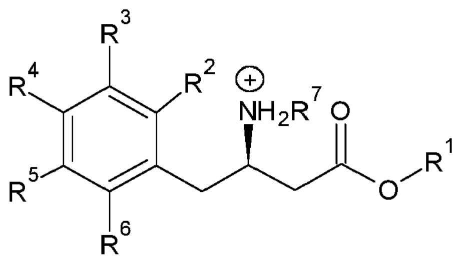

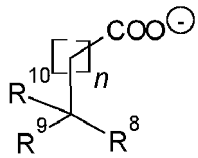

(IV)

wherein $R^1$, $R^2$, $R^3$, $R^4$, $R^5$, $R^6$, $R^7$, $R^8$, $R^9$ and $R^{10}$ have the same meaning as indicated in the embodiments

described above, wherein the salt of formula (IV) at least partially precipitates, thereby obtaining a reaction mixture depleted of compound of formula (II).

In some embodiments of the method for preparing a compound of formula (II), the method comprises (d) contacting the reaction mixture obtained in (c) with a carboxylic acid of formula (III) as defined above thereby obtaining a salt of formula (IV), which at least partially precipitates, thereby obtaining a reaction mixture depleted of compound of formula (II).

Preferably, the carboxylic acid of formula (III) comprises 9H-fluorene-9-carboxylic acid, more preferably is 9H-fluorene-9-carboxylic acid.

In some embodiments of the method for preparing a compound of formula (II), the method further comprises (e) separating the at least partially precipitated salt of formula (IV) from the reaction mixture depleted of compound of formula (II).

Additionally, the method, i.e. the biocatalytic process, can be further optimized using different formulations of the amine transaminase comprising a polypeptide having at least 80 % sequence identity with the polypeptide of SEQ ID NO. 1such as tags or immobilization. Thus, the polypeptide having at least 80 % sequence identity with the polypeptide of SEQ ID NO. 1 may be comprised in larger structures, for example, the polypeptide as specified may be comprised in fusion polypeptides comprising further peptides, which may serve for example as a tag for purification and/or detection, or as a linker. The term "detectable tag" refers to a stretch of amino acids which are added to or introduced into the fusion polypeptide; preferably, the tag is added C- or N- terminally to the fusion polypeptide of the present invention. Said stretch of amino acids preferably allows for detection of the fusion polypeptide by an antibody which specifically recognizes the tag; or it preferably allows for forming a functional conformation, such as a chelator; or it preferably allows for visualization, for example in the case of fluorescent tags. Preferred detectable tags are the Myc-tag, FLAG-tag, 6-His-tag, HA-tag, GST-tag or a fluorescent protein tag, for example a GFP-tag. These tags are all well known in the art. Furthermore, the amine transaminase comprising a polypeptide having at least 80 % sequence identity with the polypeptide of SEQ ID NO. 1 may be immobilized, for example may be covalently or non-covalently linked to carrier molecules or so called solid phases. Methods for enzyme immobilization are well known to the skilled person. The amine transaminase may also be immobilized in the form of a cross-linked enzyme aggregate (CLEA), or may be immobilized by entrapment, i.e. may be physically entrapped inside a porous matrix, wherein the bonds involved in stabilizing the enzyme to the matrix may be covalent or non-covalent. These immobilization methods are known to the skilled person in general. Process variants for carrying out the method of the present invention are for example fed-batch, fluidized bed or flow reactor technologies, which are also known to the skilled person.

2nd aspect - compound of formula (II)

In a second aspect, the present invention is directed to a compound of formula (II) obtained or obtainable from the method of the first aspect. All details described above with respect to the first aspect apply also for the second aspect.

3rd aspect - use of compound of formula (II)

A third aspect of the invention is related to the use of the compound of formula (II) obtained or obtainable from the method of the first aspect or the compound of formula (II) according to the second aspect for the preparation of sitagliptin, retagliptin or evogliptin, preferably sitagliptin. All details described above with respect to the first aspect apply also for the third aspect.

4th aspect - process for preparing sitagliptin or retagliptin or evogliptin

In a fourth aspect, the invention relates to a process for preparing sitagliptin or retagliptin or evogliptin, preferably sitagliptin, comprising

(A) providing a compound of formula (II) obtained or obtainable from the method of any one of embodiments 1 to 19 or the compound of formula (II) according to embodiment 20;
(B) optionally, if $R^1$ is not hydrogen, deprotecting the compound of formula (II) thereby obtaining a compound of formula (II) wherein $R^1$ is hydrogen;
(C) reacting the compound of formula (II) of (A) or (B) with a compound of formula (V), (VI) or (VII)

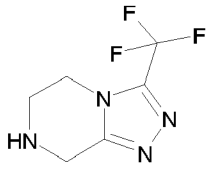

(V)

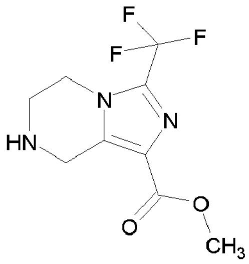

(VI)

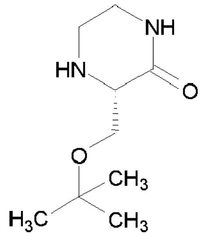

(VII)

thereby forming sitagliptin (reaction of compound of formula (II) wherein $R^1$ is hydrogen with compound of formula (V)) or retagliptin (reaction of compound of formula (II) wherein $R^1$ is hydrogen with compound of formula (VI)) or evogliptin (reaction of compound of formula (II) wherein $R^1$ is hydrogen with compound of formula (VII)). The compound of formula (V) is 3-(trifluoromethyl)-5,6,7,8-tetrahydro-[1,2,4]triazolo[4,3-a]pyrazine (CAS no 486460-21-3), the compound of formula (VI) is methyl 3-(trifluoromethyl)-5,6,7,8-tetrahydroimidazo[1,5-a]pyrazine-1-carboxylate (CAS no 1358715-37-3), compound of formula (VII) is (3R)-3-[(1,1-dimethyleth-oxy)methyl]-2-piperazinone (CAS no 1222102-49-9).

The present invention is further illustrated by the following set of embodiments and combinations of embodiments resulting from the dependencies and back-references as indicated. In particular, it is noted that in each instance where a range of embodiments is mentioned, for example in the context of a term such as "any one of embodiments (1) to (4)", every embodiment in this range is meant to be explicitly disclosed for the skilled person, i.e. the wording of this term is to be understood by the skilled person as being synonymous to "any one of embodiments (1), (2), (3), and (4)". Further, it is explicitly noted that the following set of embodiments is not the set of claims determining the extent of protection, but represents a suitably structured part of the description directed to general and preferred aspects of the present invention.

1. A method for preparing a compound of formula (II)

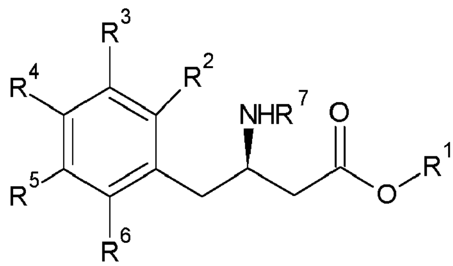

(II)

wherein

| | |
|---|---|
| $R^1$ | is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl; |
| $R^2$, $R^3$, $R^4$, $R^5$, $R^6$ | are independently of each other either a hydrogen atom or a halogen atom; |
| $R^7$ | is a hydrogen atom or a protective group; |

the method comprising:

(a) Providing a compound of formula (I)

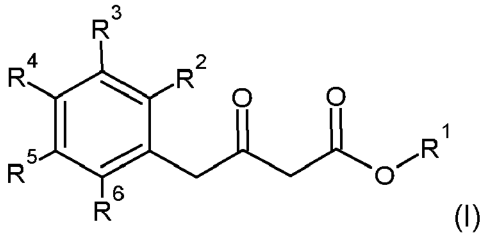

(I)

wherein $R^1$ is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl;
$R^2$, $R^3$, $R^4$, $R^5$ and $R^6$ have the same meaning as in the compound of formula (II);
(b) Providing an amine donor comprising an $-NH_2$ group;
(c) Reacting the compound of formula (I) of (a) with the amine donor of (b) in the presence of an amine transaminase, wherein the amine transaminase comprises a polypeptide having at least 80 % sequence identity with the polypeptide of SEQ ID NO. 1; thereby obtaining a reaction mixture comprising a compound of formula (II).

2. The method of embodiment 1, wherein in the compound of formula (I) and in the compound of formula (II), $R^3$, $R^4$ and $R^6$ are each a halogen atom, independently selected from the group consisting of fluorine atom, bromine atom, chlorine atom and iodine atom, preferably $R^3$, $R^4$ and $R^6$ are each the same halogen atom selected from the group consisting of fluorine atom, bromine atom, chlorine atom and iodine atom, more preferably, $R^3$, $R^4$ and $R^6$ are each a fluorine atom; and $R^2$ and $R^5$ are each a hydrogen atom.

3. The method of embodiment 1 or 2, wherein in the compound of formula (I) $R^1$ is a hydrogen atom or a C1 to C10 alkyl group, preferably a hydrogen atom or a C1 to C5 alkyl group.

4. The method of any one of embodiments 1 to 3, wherein in the compound of formula (II), $R^7$ is a hydrogen atom or a protective group selected from the group consisting of *tert*-butyloxy carbonyl (BOC), benzyloxycarbonyl (Cbz), fluorenylmethylenoxycarbonyl (Fmoc) and allyloxycarbonyl (Alloc), wherein $R^7$ is preferably a hydrogen atom or a Boc group.

5. The method of any one of embodiments 1 to 4, wherein the amine donor comprising an $-NH_2$ group is selected from the group consisting of ethylamine, isopropylamine, butylamine, L-alanine, , L-phenylalanine, , L-glutamine, L-leucine, 3-aminobutyric acid, 1-phenylethanamine, (*S*)-phenylethanamine, 2-amino-4-phenylbutane, glycine, L-glutamic acid, , L-aspartic acid, L-lysine, L-ornithine, beta-alanine, taurine, n-octylamine, cyclohexylamine, 1,4-butanediamine, 1,6-hexanediamine, 6-amino hexanoic acid, 4-aminobutyric acid, tyramine, benzyl amine, 2-aminobutane, 2- amino-1-butanol, 1-amino-1-phenylethane, 1-amino-1-(2-methoxy-5-fluorophenyl) ethane, 1-amino-1-phenylpropane, 1-amino-L-(4-hydroxyphenyl) propane, 1-amino-1-(4-bromophenyl)propane, 1-amino-1-(4-nitrophenyl)propane, 1-phenyl-2-amino propane, L-(3-trifluoromethylphenyl)-2-aminopropane, 2-aminopropanol, 1-amino-1-phenylbutane, 1-phenyl-2-aminobutane, 1-(2,5-di-methoxy-4-methylphenyl)-2-amino butane, L-phenyl-3-aminobutane, L-(4-hydroxyphenyl)-3-aminobutane, L-amino-2- methylcyclopentane, L-amino-3-methylcyclopentane, L-amino-2-methylcyclohexane, 1-amino-1-(2-naphthyl)ethane, 3-methylcyclopentylamine, 2-methylcyclo pentylamine, 2-ethylcyclopentylamine, 2-methylcyclohexylamine, 3-methylcyclohexylamine, 1-aminotetralin, 2-aminotetralin, 2-amino-5-methoxytetralin, and 1-aminoindane, L-threonine, L-cysteine, tryptophan, serine, phenylglycinol, tyrosine, glutamine, methionine, 5-amino valeric, 8-amino caproic acid, 2-aminobutaric acid, D-2-galactosamine, ethyl 3-aminobutyrate, 3-amino-3-phenylpropionic acid, mixtures of two or more of these amine donors, the racemates of these amine donors, salts of these amine donors or of their racemates, and mixtures of one or more of these amine donors with one or more salt(s) of these amine donors, wherein the amine donor comprising an $-NH_2$ group is preferably selected from the group consisting of isopropylamine, L-alanine, , phenylalanine, glutamine, 3-aminobutyric acid, 2-amino-4-phenylbutane, L-glutamic acid, L-aspartic acid, beta-alanine, 6-amino hexanoic acid, 5-amino valeric acid, 8-amino caproic acid, 4-aminobutyric acid, 2-aminobutaric acid, 1-phenyl-2-amino propane, L-(3-trifluoromethylphenyl)-2-aminopropane, L-phenyl-3-aminobutane, L-(4-hydroxyphenyl)-3-aminobutane, D-2-ga-lactosamine, ethyl 3-aminobutyrate, 3-amino-3-phenylpropionic acid, mixtures of two or more of these amine donors, salts of these amine donors, and mixtures of one or more of these amine donors with one or more salt(s) of these amine donors, wherein the amine donor comprising an $-NH_2$ group is more preferably selected from L-alanine, L-aspartic acid mixtures of two or more of these amine donors, the racemates of these amine donors, salts of these amine donors or of their racemates, and mixtures of one or more of these amine donors with one or more salt(s) of these amine donors and/or with one or more of their racemates, more preferably the amine donor comprising

an $-NH_2$ group is selected from L-alanine, racemate comprising L-alanine, L-aspartic acid, racemate comprising L-aspartic acid, a mixture of L-alanine and L-aspartic acid, mixture of racemate comprising L-alanine and L-aspatic acid, mixture of L-alanine with racemate comprising L-aspartic acid, salts of L-alanine, salts of racemate comprising L-aspartic acid, salts of L-aspartic acid, salts of racemate comprising L-aspartic acid, mixtures of L-alanine with one or more salt(s) of L-aspartic acid, mixtures of racemate comprising L-alanine with one or more salt(s) of L-aspartic acid, mixtures of one or more salt(s) of L-alanine with L-aspartic acid and mixtures of one or more salt(s) of L-alanine with racemate comprising L-aspartic acid.

6. The method of any one of embodiments 1 to 5, wherein reacting in (c) is done at a temperature in the range of from 10 to 55 °C, preferably in the range of from 15 to 45°C, more preferably in the range of from 20 to 40 °C, more preferably in the range of from 25 to 30°C.

7. The method of any one of embodiments 1 to 6, wherein reacting in (c) is done at a pressure in the range of from 20 mbar to 2 bar, preferably in the range of from 100 mbar to 1.5 bar, more preferably in the range of from 800 to 1300 mbar, more preferably in the range of from 900 to 1100 mbar.

8. The method of any one of embodiments 1 to 7, wherein reacting in (c) is done in solution, preferably in aqueous solution, wherein the aqueous solution preferably comprises at least 60 weight-%, more preferably at least 80 weight-%, more preferably at least 95 weight-%, more preferably at least 98 weight-%, water, based on the overall weight of the aqueous solution.

9. The method of embodiment 8, wherein the aqueous solution comprises an organic solvent, wherein the aqueous solution preferably comprises at the outmost 40 weight-%, more preferably at the outmost 20 weight-%, more preferably at the outmost 5 weight-%, more preferably a the outmost 2 weight-% organic solvent, based on the overall weight of the aqueous solution, wherein the organic solvent is preferably a polar organic solvent having an n-octanol/Water partition coefficient (Kow) in the range of from 1 to 25, preferably in the range of from 2 to 22, at 298 K, preferably the organic solvent is selected from the group consisting of dimethyl sulfoxide (DMSO, Kow = 2), methanol (Kow = 19), ethanol (Kow = 22) 2,2,2- trifluoroethanol (Kow = 2), N,N-Dimethylformamide (DMF, Kow = 4), ethylene glycol (1,2-ethandiol, $K_{OW}$ = 4), propylene glycol (1,2-propane diol, Kow = 4), and mixtures of two or more of these solvents.

10. The method of any one of embodiments 1 to 8, wherein reacting in (c) is done in buffered solution, wherein the buffer is preferably selected from the group of (4-(2-hydroxyethyl)-1-piperazineethanesulfonic acid) buffer (HEPES), N-cyclohexyl-2-aminoethane sulfonic acid buffer (CHES), sodium phosphate buffer (Napp), potassium phosphate buffer (Kapp), tris(hydroxymethyl)aminomethane hydrochloride buffer (Tris-HCI), buffer comprising a mixture of tris base, acetic acid and EDTA (TAE), 3-(N-morpholino)propanesulfonic acid buffer (MOPS), borate buffered saline (BBS), phosphate-buffered saline (PBS) and mixtures of two or more of these buffers, more preferably a buffer selected from the group of Napp, HEPES, CHES, CAPS and mixtures of two or more of these buffers, more preferably a buffer selected from the group of Napp, HEPES, CHES and mixtures of two or more of these buffers, more preferably a buffer selected from the group of HEPES, CHES and mixtures of these buffers, more preferably the buffer comprises at least CHES.

11. The method of any one of embodiments 1 to 10, wherein reacting in (c) is done at a pH value in the range of from 6 to 11, preferably in the range of from 8 to 11, more preferably in the range of from 9 to 10.5, more preferably in the range of from 9.75 to 10.25.

12. The method of any one of embodiments 1 to 11, wherein reacting in (c) is done at a concentration of the compound of formula (I) in the range of from 0.1 to 1000 mM, preferably in the range of from 1 to 200 mM, more preferably in the range of from 3 to 90 mM, more preferably in the range of from 5 to 80 mM.

13. The method of any one of embodiments 1 to 12, wherein reacting in (c) is done with a ratio of the amine donor of (b) to the compound of formula (I) in the range of from 1:1 to 100:1, preferably in the range of from 1:1 to 50:1, more preferably 1:1 to 30:1, more preferably in the range of from 1:1 to 25:1.

14. The method of any one of embodiments 1 to 13, wherein reacting in (c) is done in the presence of a cofactor, preferably pyridoxal 5'-phosphate (PLP), wherein the cofactor, preferably PLP, preferably has a concentration in the range of from 0.01-5 mM.

15. The method of any one of embodiments 1 to 14, wherein reacting in (c) is done in the presence of a further enzyme, preferably selected from the group consisting of lactate dehydrogenase, glucose dehydrogenase, alanine dehydrogenase, formate dehydrogenase and a mixture of at least two of these further enzymes (equilibrium shift by enzyme cascades).

16. The method of any one of embodiments 1 to 15, wherein reacting in (c) is done in the presence of a carboxylic acid of formula (III)

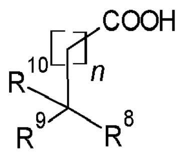
(III)

wherein:

n is zero or 1;
the residues $R^8$ and $R^9$ together form a fluorine ring or are both phenyl or together form a phenyl ring, wherein each phenyl ring has at least one further substituent selected from the group consisting of hydrogen atom, halogen atom, and nitro group;
and the residue $R^{10}$ is hydrogen atom or methyl or absent if $R^8$ and $R^9$ together form a phenyl ring; thereby obtaining a salt of formula (IV)

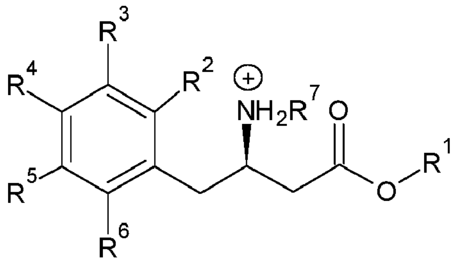

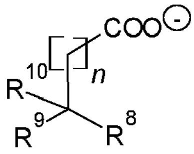
(IV)

wherein $R^1$, $R^2$, $R^3$, $R^4$, $R^5$, $R^6$, $R^7$, $R^8$, $R^9$ and $R^{10}$ have the same meaning as indicated in embodiments 1 to 14, wherein the salt of formula (IV) at least partially precipitates, thereby obtaining a reaction mixture depleted of compound of formula (II).

17. The method of any one of embodiments 1 to 16, preferably 1 to 15, comprising (d) contacting the reaction mixture obtained in (c) with a carboxylic acid of formula (III) as defined in embodiment 16, thereby obtaining a salt of formula (IV), which at least partially precipitates, thereby obtaining a reaction mixture depleted of compound of formula (II).

18. The method of embodiment 16 or 17, wherein the carboxylic acid of formula (III) comprises 9H-fluorene-9-carboxylic acid, preferably is 9H-fluorene-9-carboxylic acid.

19. The method of any one of embodiments 16 to 18 comprising:
(e) separating the at least partially precipitated salt of formula (IV) from the reaction mixture depleted of compound of formula (II).

20. The method of any one of embodiments 1 to 19, wherein the amine transaminase of (c) comprises a polypeptide having at least 80 % sequence identity, preferably at least 85 % sequence identity, more preferably at least 90 %

sequence identity, more preferably at least 95 % sequence identity, more preferably at least 98 % sequence identity, more preferably at least 99 % sequence identity, more preferably at least 99.5 % sequence identity, with the polypeptide of SEQ ID NO. 1, wherein more preferably the amine transaminase of (c) consists of a polypeptide having at least 85 % sequence identity, preferably at least 90 % sequence identity, more preferably at least 95 % sequence identity, more preferably at least 98 % sequence identity, more preferably at least 99 % sequence identity, more preferably at least 99.5 % sequence identity, with the polypeptide of SEQ ID NO. 1.

21. A compound of formula (II) obtained or obtainable from the method of any one of embodiments 1 to 20.

22. Use of the compound of formula (II) obtained or obtainable from the method of any one of embodiments 1 to 20 or the compound of formula (II) according to embodiment 21 for the preparation of sitagliptin, retagliptin or evogliptin, preferably sitagliptin.

23. A process for preparing sitagliptin or retagliptin or evogliptin, preferably sitagliptin, comprising

(A) providing a compound of formula (II) obtained or obtainable from the method of any one of embodiments 1 to 20 or the compound of formula (II) according to embodiment 21;
(B) optionally, if $R^1$ is not hydrogen, deprotecting the compound of formula (II) thereby obtaining a compound of formula (II) wherein $R^1$ is hydrogen;
(C) reacting the compound of formula (II) of (A) or (B) with a compound of formula (V), (VI) or (VII)

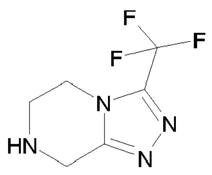

(V)

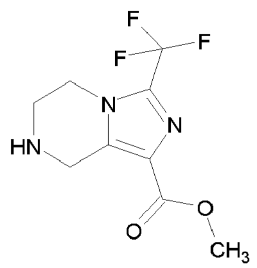

(VI)

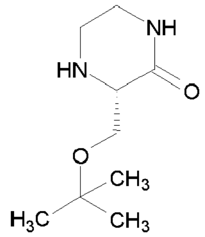

(VII)

thereby forming sitagliptin (reaction of formula (II) wherein $R^1$ is hydrogen with formula (V)) or retagliptin (reaction of formula (II) wherein $R^1$ is hydrogen with formula (VI)) or evogliptin (reaction of formula (II) wherein $R^1$ is hydrogen with formula (VII)).

The present invention is further illustrated by the following reference examples, comparative examples, and examples.

**Examples**

**Reference Example 1: Instrumental analytics method of the biocatalysis reactions**

In a biocatalysis reaction, the product was detected via GC-MS after derivatization with methylchloroformate.
The biocatalysis reaction was terminated by adding 20 μl of 10 M NAOH to 180 μl of reaction mixture. Following which, 167 μl of methanol, 34 μl of pyridine and 40 μl of methylchloroformate was added for the derivatization of the product amine. 20 mM of phenylacetic acid was added as an internal standard. After derivatization the sample was extracted with 100 μl of chloroform followed by washing with 100 μl 100 mM sodium bi-carbonate. The organic phase was dried with magnesium sulphate and analyzed via GC using Hydrodex β-TBDAc column. The derivatization and extraction of the (*R*)-3-amino-4-(2,4,5-trifluorophenyl)butanoate standard was performed at different concentrations (0.2 mM, 0.4 mM, 0.6 mM, 0.8 mM, and 1 mM) with 20 mM of phenylacetic acid as an internal standard for the quantification. (*R*)-3-amino-4-(2,4,5-trifluorophenyl)butanoate and its methyl ester both yield the same derivative and thus are quantified together.

**Reference Example 2: Enzyme preparation and purification**

The gene encoding the putative transaminase comprising a polypeptide of SEQ ID NO. 1 is part of an expression vector, for example pET28b, having one or more control sequences for the expression of the transaminase. The expression vector comprising the gene encoding for the putative transaminase, is introduced into *E. coli* BL21 (DE3) cells. The *E. coli* BL21 (DE3) cells transformed with the expression vector were grown in Terrific Broth medium (TB-medium) supplemented with 50 μg ml-1 kanamycin (pET28b) and induction was carried out at an optical density (OD600) of about 1.0 with 1 mM isopropyl-D-thiogalactopyranoside (IPTG) and the culture was shaken at 20°C for 16-20 h and then centrifuged for harvesting the cells. The cell pellet was washed with 40 ml of lysis buffer (Buffer-A, sodium phosphate 50 mM, pH 8.0) and then resuspended in 20 ml of loading buffer (Buffer-B, sodium phosphate 50 mM, pH 8.0, containing 0.3 M NaCl, and 0.1 mM pyridoxal 5'-phosphate, PLP). After disruption by sonication at 0°C for 10 min, the suspension was centrifuged at (8500 x g, 1 h) and the supernatant was passed through a 0.45 μm filter prior to chromatography. Chromatography was performed using an Äkta Purifier. As the recombinant proteins contained a His6-tag, a 5 ml Ni-NTA (nickel nitrilotriacetic acid) column (GE Health care) was used for purification. After washing the column with 60 ml of binding buffer (Buffer-C, sodium phosphate 50 mM, pH 8.0; containing 0.3 M NaCl, 0.1 mM PLP, and 0.03 M imidazole) the crude extract was loaded. Enzyme was eluted by elution buffer (Buffer-D, sodium phosphate 50 mM, pH 8.0; containing 0.3 M NaCl, 0.3 M imidazole, and 0.1 mM PLP; 5 ml min-1 flow rate) and the fractions containing the desired protein were collected. For desalting, size exclusion chromatography with 5 ml Sephadex desalting columns (GE Healthcare) using Buffer-A was performed. The expression efficiency of the right enzyme was verified using sodium dodecyl sulfate-polyacrylamide gel electrophoresis (SDS-PAGE). The resulting enzyme was a transaminase comprising an amino acid sequence according to SEQ ID NO. 1 (UniProt: E1V8W4, Genebank accession number MT828898); here the transaminase contained a C terminal His6-tag and had an amino acid sequence according to SEQ ID NO. 2 (amino acid sequence according to SEQ ID NO. 1 + 6x histidine at the C terminus).

**Reference Example 3: Biocatalysis**

Reactions were performed at 1 ml scale with 10 mM substrate concentration of methyl 3-oxo-4-(2, 4, 5-trifluorophenyl) butanoate, 100 mM amine donor (L-alanine), 100 mM sodium phosphate buffer ((Napp, pH 8.0) and an enzyme final concentration of 1 mg/ml. The reaction vials were incubated at 30 °C for 24 hours. The educt was methyl-3-amino-4-(2, 4, 5-trifluorophenyl) butanoate. The conversion was defined as the formed molar amount of derivatized product in relation to the initial molar amount of educt at the beginning of the reaction, expressed in percentage by the following equitation (1):

$$(1) \quad C = \frac{m_p - m_e}{m_e} \times 100\%$$

wherein $m_p$ was the molar amount of formed product and $m_e$ was the initial molar amount of educt at the beginning of the reaction.

**Example 1 - Optimization of pH**

For determining the pH optima, biocatalysis was carried out according to Reference Example 3 at different pH (7-8) sodium phosphate buffer (Napp or HEPES), (9-10), CHES buffer (10-11) CAPS buffer. Reactions were performed with an amino donor, L-alanine, concentration of 100 mM, a substrate (methyl 3-oxo-4-(2, 4, 5-trifluorophenyl) butanoate) loading of 10 mM and enzyme 1mg/ml at 30 °C. The buffer, pH value and the resulting conversion in percent are summarized in Table 1 below:

**Table 1**

| Variation of conversion with different pH and buffer | | |
|---|---|---|
| Buffer | pH | Conversion [%] |
| Napp | 8 | 3,5 |
| HEPES | 8 | 3 |
| Ches | 9 | 4,4 |
| Ches | 10 | 8 |

(continued)

| Variation of conversion with different pH and buffer | | |
|---|---|---|
| Buffer | pH | Conversion [%] |
| Caps | 10,5 | 5 |
| Caps | 11 | 4 |

It was found that a reaction at a pH in the range of from 8 to 11 gave acceptable results, wherein a biocatalysis reaction at about pH 10 gave the best conversion.

**Example 2 - Optimization of temperature**

Effect of temperature on the biocatalytic conversion was studied by performing the biocatalysis reaction according to Reference Example 3 at 20 °C, 30 °C and 40 °C. Reactions were performed in 100 mM CHES buffer (pH 9 or 10), with L-alanine as amino donor, concentration of 100 mM, a substrate (methyl 3-oxo-4-(2, 4, 5-trifluorophenyl) butanoate) loading of 10 mM and enzyme 1mg/ml at different temperature. The pH, temperature and the resulting conversion in percent are summarized in Table 2 below:

**Table 2**

| Variation of conversion with different temperature | | |
|---|---|---|
| pH | Temperature [°C] | Conversion [%] |
| 9 | 25 | 4 |
| | 30 | 3,8 |
| | 40 | 0,6 |
| 10 | 25 | 7 |
| | 30 | 8 |
| | 40 | 1 |

It was found that reaction temperatures in the range of from 25 to 40 °C gave acceptable conversion results, wherein a reaction at a temperature in the range of from 25 to 30°C gave the best conversion.

**Example 3 - Investigation of amino donors**

In addition to L-alanine, several other amino compounds (glutamic acid, aspartic acid, cysteine, beta-alanine, L-theronine) were tested as putative amino donors in a biocatalysis according to Reference Example 3. Reactions were performed in 100 mM CHES buffer (pH 10), with an amino donor concentration of 100 mM, a substrate (Methyl 3-oxo-4-(2, 4, 5-trifluorophenyl) butanoate) loading of 10 mM and enzyme 1mg/ml at 30 °C. The amine donors and the resulting conversion in percent are summarized in Table 3 below:

**Table 3**

| Variation of conversion with different amine donor | |
|---|---|
| Amine donor | Conversion [%] |
| L-alanine | 8 |
| L-glutamic acid | 18 |
| beta-alanine | 8 |
| L-cysteine | 3 |
| L-aspartic acid | 30 |

(continued)

| Variation of conversion with different amine donor | |
|---|---|
| Amine donor | Conversion [%] |
| L-threonine | 3 |

Very unexpected is the finding that L-aspartate was found to increase the conversion to 30 %. Furthermore, L-alanine, beta alanine and L-glutamic acid gave good conversion results.

**Example 4 - Investigation of amino donors' concentration**

Given the equilibrium-controlled transaminase reaction, different concentrations of amin donor were tested in the biocatalysis according to Reference Example 3. Reactions were performed in 100 mM CHES buffer (pH 10), with an amine donor concentration of 100 mM, 200 mM and 300 mM, along with a substrate (methyl 3-oxo-4-(2, 4, 5-trifluorophenyl) butanoate) loading of 10 mM and enzyme 1mg/ml at 30 °C. The amine donors, their concentration and the resulting conversion are summarized in Table 4 below:

**Table 4**

| Variation of conversion with different amine donor concentration | | |
|---|---|---|
| Amine donor | Amine donor concentration [mM] | Conversion [%] |
| L-alanine | 100 | 19 |
| | 200 | 16 |
| L-glutamic acid | 100 | 30 |
| | 200 | 32 |
| L-aspartic acid | 100 | 38 |
| | 200 | 49 |
| | 300 | 45 |

It was found that an amine donor concentration on the range of from 100 to 300 mM gave good conversion results.

**Example 4 - Crystallization for product isolation**

Crystallisation of the amine product - here methyl (*R*)-3-amino-4-(2, 4, 5-trifluorophenyl) butanoate - was performed by acid addition, thus forming an acid salt comprising a carboxylate anion and the amine product with a positive charge at the N-atom as cation.

The first step to screen for the acid salt was performed by using the Organic acid Screening Kit, by Enzymicals AG, consisting of 96 most potential organic acid for *in situ* precipitation of amine products.

All 96 separate 400 mM acid solutions (typically 20 ml) were prepared by dissolving the respective acid in 50 mM phosphate buffer pH 7.5. Afterwards within all resulting solutions the pH was carefully adjusted back to 7.5. Please note that acids with low aqueous solubility sometimes required multiple pH adjustments due to an additional dissolution process after its previous pH-adjustment. Acid solution with a remaining solid fraction were filtered and used in their resulting (unknown) concentration.

The amine product was dissolved in 50 mM phosphate buffer pH 7.5 with a concentration of 100 mM and the pH was adjusted to 7.5 in order to have a neutralized solution of the amine product. 200 ml fractions of the neutralized acid solutions (96 in total) were given each into a 96 well plate and their position documented. Afterwards 200 ml of neutralized amine product solution was added into each filled well, which led to a clear solution or an almost instantaneous precipitation. The result was documented by visual observation and photography against a black background after 1 and 24 h.

Fluoren-9-carboxylic acid was found to be able to form a precipitable acid salt.

**Example 5 - PLP Assay**

Transaminases are pyridoxal-5'-phosphate (PLP)-dependent enzymes. Hence, the PLP depletion over time was used as a standard procedure to determine the chemical substrate acceptance/product formation by a given transaminase. PLP assay was performed at 30 °C, in 200 μl volume with buffer (50 mM Ches, pH 10), 50 mM substrate, and 50 μl of the purified enzyme having a concentration of 5 mg/ml (as obtained by Reference Example 2). The bound PLP consumption was monitored by measuring the absorbance at 410 nm.

It was observed a rapid decrease of absorbance at 410 nm within seconds indicating a PLP depletion with a compound of formula IIa (*R*)-3-amino-4-(2, 4, 5-trifluorophenyl) butanoic acid and IIb methyl-(*R*)-3-amino-4-(2, 4, 5-trifluorophenyl) butanoate, while with the analoguous compounds with (S)-configuration a significant change of absorbance was not observed, indicating that the enzyme did not convert the (S)-configured analogues. With (*R*)-3-amino-4-(2, 4, 5-trifluorophenyl) butanoic acid and methyl-(*R*)-3-amino-4-(2, 4, 5-trifluorophenyl) butanoate the enzyme showed an activity of 0.7 U/g protein. This indicated the enantio-preference of the transaminase for the production of (*R*)-ester and/or (*R*)-acid of formula IIa / IIb.

Definition of unit and explanation of calculation:
1U is defined as 1 μmol of PLP consumption in a minute and is calculated by the following equation (2):

$$(1)\quad activity\ U/g = \frac{\frac{\Delta A}{\Delta t} * d}{\varepsilon}$$

Wherein, ($\Delta A/\Delta t$) is the slope showing the rate of change of absorbance at 410 nm, d is the dilution factor and ε is the extinction co-efficient. Here, an ε was determined to be 181 mM-1 and used thereafter for all calculation.

**Cited Literature**

- Savile et al., Science 2010, 329, 30
- IN 1391CHE2014
- CN 104805069 B
- CN 106801043 A
- CN 106191148 A
- WO 2019/128894 A1
- WO 2019/007146A1
- CN 105331651 A
- Kim et al, Enzyme Microb. Technol. 2019, 120, 52
- Hou et al, Tetrahedron 2016, 72, 4660
- Busto et al., Adv. Synth. Catal. 2014, 356, 1937


**Claims**

**1.** A method for preparing a compound of formula (II)

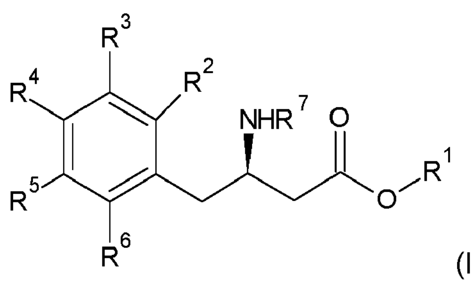

(II)

wherein

$R^1$ is selected from the group consisting of hydrogen atom, C1 to C10 alkyl, C6 to C12 aryl, C1 to C5 alkyl-C6

to C12 aryl, C3 to C10 heteroaryl, and C1 to C5 alkyl-C3 to C10 heteroaryl;
$R^2$, $R^3$, $R^4$, $R^5$, $R^6$ are independently of each other either a hydrogen atom or a halogen atom;
$R^7$ is a hydrogen atom or a protective group;

the method comprising:

(a) Providing a compound of formula (I)

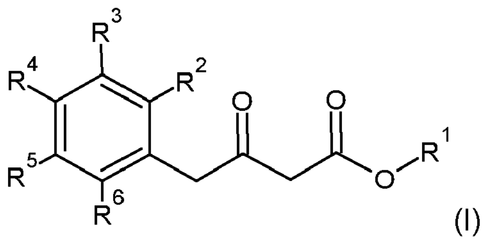

(I)

wherein $R^1$ is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl;
$R^2$, $R^3$, $R^4$, $R^5$ and $R^6$ have the same meaning as in the compound of formula (II);
(b) Providing an amine donor comprising an $-NH_2$ group;
(c) Reacting the compound of formula (I) of (a) with the amine donor of (b) in the presence of an amine transaminase, wherein the amine transaminase comprises a polypeptide having at least 80 % sequence identity with the polypeptide of SEQ ID NO. 1; thereby obtaining a reaction mixture comprising a compound of formula (II).

**2.** The method of claim 1, wherein in the compound of formula (I) and in the compound of formula (II), $R^3$, $R^4$ and $R^6$ are each a halogen atom, independently selected from the group consisting of fluorine atom, bromine atom, chlorine atom and iodine atom, preferably $R^3$, $R^4$ and $R^6$ are each the same halogen atom selected from the group consisting of fluorine atom, bromine atom, chlorine atom and iodine atom, more preferably, $R^3$, $R^4$ and $R^6$ are each a fluorine atom; and $R^2$ and $R^5$ are each a hydrogen atom; and/or

wherein in the compound of formula (I) $R^1$ is a hydrogen atom or a C1 to C10 alkyl group, preferably a hydrogen atom or a C1 to C5 alkyl group;
and/or
wherein in the compound of formula (II), $R^7$ is a hydrogen atom or a protective group selected from the group consisting of *tert* butyloxy carbonyl (BOC), benzyloxycarbonyl (Cbz), fluorenylmethylenoxycarbonyl (Fmoc) and allyloxycarbonyl (Alloc), wherein $R^7$ is preferably a hydrogen atom or a Boc group.

**3.** The method of claim 1 or 2, wherein the amine donor comprising an $-NH_2$ group is selected from the group consisting of ethylamine, isopropylamine, butylamine, L-alanine, , L-phenylalanine, , L-glutamine, L-leucine, 3-aminobutyric acid, 1-phenylethanamine, (5)-phenylethanamine, 2-amino-4-phenylbutane, glycine, L-glutamic acid, , L-aspartic acid, L-lysine, L-ornithine, beta-alanine, taurine, n-octylamine, cyclohexylamine, 1,4-butanediamine, 1,6-hexanediamine, 6-amino hexanoic acid, 4-aminobutyric acid, tyramine, benzyl amine, 2-aminobutane, 2- amino-1-butanol, 1-amino-1-phenylethane, 1-amino-1-(2-methoxy-5-fluorophenyl) ethane, 1-amino-1-phenylpropane, 1-amino-L-(4-hydroxyphenyl) propane, 1-amino-1-(4-bromophenyl)propane, 1-amino-1-(4-nitrophenyl)propane, 1-phenyl-2-amino propane, L-(3-trifluoromethylphenyl)-2-aminopropane, 2-aminopropanol, 1-amino-1-phenylbutane, 1-phenyl-2-aminobutane, 1-(2,5-dimethoxy-4-methylphenyl)-2-amino butane, L-phenyl-3-aminobutane, L-(4-hydroxyphenyl)-3-aminobutane, L-amino-2- methylcyclopentane, L-amino-3-methylcyclopentane, L-amino-2-methylcyclohexane, 1-amino-1-(2-naphthyl)ethane, 3-methylcyclopentylamine, 2-methylcyclo pentylamine, 2-ethylcyclopentylamine, 2-methylcyclohexylamine, 3-methylcyclohexylamine, 1-aminotetralin, 2-aminotetralin, 2-amino-5-methoxytetralin, and 1-aminoindane, L-threonine, L-cysteine, tryptophan, serine, phenylglycinol, tyrosine, glutamine, methionine, 5-amino valeric, 8-amino caproic acid, 2-aminobutaric acid, D-2-galactosamine, ethyl 3-aminobutyrate, 3-amino-3-phenylpropionic acid, mixtures of two or more of these amine donors, the racemates of these amine donors, salts of these amine donors or of their racemates, and mixtures of one or more of these amine donors with one or more salt(s) of these amine donors, wherein the amine donor comprising an $-NH_2$ group is preferably selected from the group consisting of isopropylamine, L-alanine, , phenylalanine, glutamine, 3-aminobutyric acid, 2-amino-4-phenylbutane, L-glutamic acid, L-aspartic acid, beta-alanine, 6-amino hexanoic acid, 5-amino valeric acid, 8-amino caproic acid, 4-aminobutyric acid, 2-aminobutaric acid, 1-phenyl-2-amino propane, L-(3-trifluoromethylphenyl)-2-aminopropane,

L-phenyl-3-aminobutane, L-(4-hydroxyphenyl)-3-aminobutane, D-2-galactosamine, ethyl 3-aminobutyrate, 3-amino-3-phenylpropionic acid, mixtures of two or more of these amine donors, salts of these amine donors, and mixtures of one or more of these amine donors with one or more salt(s) of these amine donors, wherein the amine donor comprising an -$NH_2$ group is more preferably selected from L-alanine, L-aspartic acid mixtures of two or more of these amine donors, the racemates of these amine donors, salts of these amine donors or of their racemates, and mixtures of one or more of these amine donors with one or more salt(s) of these amine donors and/or with one or more of their racemates, more preferably the amine donor comprising an -$NH_2$ group is selected from L-alanine, racemate comprising L-alanine, L-aspartic acid, racemate comprising L-aspartic acid, a mixture of L-alanine and L-aspartic acid, mixture of racemate comprising L-alanine and L-aspatic acid, mixture of L-alanine with racemate comprising L-aspartic acid, salts of L-alanine, salts of racemate comprising L-aspartic acid, salts of L-aspartic acid, salts of racemate comprising L-aspartic acid, mixtures of L-alanine with one or more salt(s) of L-aspartic acid, mixtures of racemate comprising L-alanine with one or more salt(s) of L-aspartic acid, mixtures of one or more salt(s) of L-alanine with L-aspartic acid and mixtures of one or more salt(s) of L-alanine with racemate comprising L-aspartic acid.

**4.** The method of any one of claims 1 to 3, wherein reacting in (c) is done at a temperature in the range of from 10 to 55 °C, preferably in the range of from 15 to 45°C, more preferably in the range of from 20 to 40 °C, more preferably in the range of from 25 to 30°C;
and/or
wherein reacting in (c) is done at a pressure in the range of from 20 mbar to 2 bar, preferably in the range of from 100 mbar to 1.5 bar, more preferably in the range of from 800 to 1300 mbar, more preferably in the range of from 900 to 1100 mbar.

**5.** The method of any one of claims 1 to 4, wherein reacting in (c) is done in solution, preferably in aqueous solution, wherein the aqueous solution preferably comprises at least 60 weight-%, more preferably at least 80 weight-%, more preferably at least 95 weight-%, more preferably at least 98 weight-%, water, based on the overall weight of the aqueous solution; wherein the aqueous solution preferably comprises an organic solvent, wherein the aqueous solution preferably comprises at the outmost 40 weight-%, more preferably at the outmost 20 weight-%, more preferably at the outmost 5 weight-%, more preferably a the outmost 2 weight-% organic solvent, based on the overall weight of the aqueous solution, wherein the organic solvent is preferably a polar organic solvent having an n-octanol/Water partition coefficient (Kow) in the range of from 1 to 25, preferably in the range of from 2 to 22, at 298 K, preferably the organic solvent is selected from the group consisting of dimethyl sulfoxide (DMSO, Kow = 2), methanol (Kow = 19), ethanol (Kow = 22) 2,2,2- trifluoroethanol (Kow = 2), N,N-dimethylformamide (DMF, Kow = 4), ethylene glycol (1,2-ethandiol, Kow = 4), propylene glycol (1,2-propane diol, Kow = 4), and mixtures of two or more of these solvents.

**6.** The method of any one of claims 1 to 5, wherein reacting in (c) is done in buffered solution, wherein the buffer is preferably selected from the group of (4-(2-hydroxyethyl)-1-piperazineethanesulfonic acid) buffer (HEPES), N-cyclohexyl-2-aminoethane sulfonic acid buffer (CHES), sodium phosphate buffer (Napp), potassium phosphate buffer (Kapp), tris(hydroxymethyl)aminomethane hydrochloride buffer (Tris-HCl), buffer comprising a mixture of tris base, acetic acid and EDTA (TAE), 3-(N-morpholino)propanesulfonic acid buffer (MOPS), borate buffered saline (BBS), phosphate-buffered saline (PBS) and mixtures of two or more of these buffers, more preferably a buffer selected from the group of Napp, HEPES, CHES, CAPS and mixtures of two or more of these buffers, more preferably a buffer selected from the group of Napp, HEPES, CHES and mixtures of two or more of these buffers, more preferably a buffer selected from the group of HEPES, CHES and mixtures of these buffers, more preferably the buffer comprises at least CHES.

**7.** The method of any one of claims 1 to 6, wherein reacting in (c) is done at a pH value in the range of from 6 to 11, preferably in the range of from 8 to 11, more preferably in the range of from 9 to 10.5, more preferably in the range of from 9.75 to 10.25.

**8.** The method of any one of claims 1 to 7, wherein reacting in (c) is done at a concentration of the compound of formula (I) in the range of from 0.1 to 1000 mM, preferably in the range of from 1 to 200 mM, more preferably in the range of from 3 to 90 mM, more preferably in the range of from 5 to 80 mM.

**9.** The method of any one of claims 1 to 8, wherein reacting in (c) is done with a ratio of the amine donor of (b) to the compound of formula (I) in the range of from 1:1 to 100:1, preferably in the range of from 1:1 to 50:1, more preferably 1:1 to 30:1, more preferably in the range of from 1:1 to 25:1.

**10.** The method of any one of claims 1 to 9, wherein reacting in (c) is done in the presence of a carboxylic acid of formula (III)

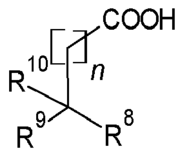

(III)

wherein:

n is zero or 1;
the residues $R^8$ and $R^9$ together form a fluorine ring or are both phenyl or together form a phenyl ring, wherein each phenyl ring has at least one further substituent selected from the group consisting of hydrogen atom, halogen atom, and nitro group;
and the residue $R^{10}$ is hydrogen atom or methyl or absent if $R^8$ and $R^9$ together form a phenyl ring; thereby obtaining a salt of formula (IV)

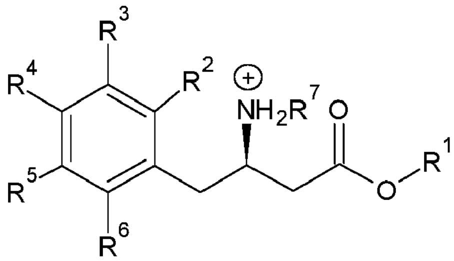

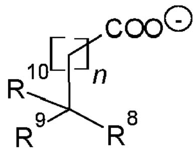

(IV)

wherein $R^1$, $R^2$, $R^3$, $R^4$, $R^5$, $R^6$, $R^7$, $R^8$, $R^9$ and $R^{10}$ have the same meaning as indicated in claims 1 to 14, wherein the salt of formula (IV) at least partially precipitates,
thereby obtaining a reaction mixture depleted of compound of formula (II).

**11.** The method of any one of claims 1 to 10 comprising

(d) contacting the reaction mixture obtained in (c) with a carboxylic acid of formula (III) as defined in claim 10, thereby obtaining a salt of formula (IV), which at least partially precipitates, thereby obtaining a reaction mixture depleted of compound of formula (II);
wherein the carboxylic acid of formula (III) preferably comprises 9H-fluorene-9-carboxylic acid, more preferably is 9H-fluorene-9-carboxylic acid;
and optionally
(e) separating the at least partially precipitated salt of formula (IV) from the reaction mixture depleted of compound of formula (II).

**12.** The method of any one of claims 1 to 11, wherein the amine transaminase of (c) comprises a polypeptide having at least 80 % sequence identity, preferably at least 85 % sequence identity, more preferably at least 90 % sequence identity, more preferably at least 95 % sequence identity, more preferably at least 98 % sequence identity, more preferably at least 99 % sequence identity, more preferably at least 99.5 % sequence identity, with the polypeptide of SEQ ID NO. 1, wherein more preferably the amine transaminase of (c) consists of a polypeptide having at least 85 % sequence identity, preferably at least 90 % sequence identity, more preferably at least 95 % sequence identity, more preferably at least 98 % sequence identity, more preferably at least 99 % sequence identity, more preferably at least 99.5 % sequence identity, with the polypeptide of SEQ ID NO. 1.

**13.** A compound of formula (II) obtained or obtainable from the method of any one of claims 1 to 12.

**14.** Use of the compound of formula (II) obtained or obtainable from the method of any one of claims 1 to 12 or the compound of formula (II) according to claim 13 for the preparation of sitagliptin, retagliptin or evogliptin, preferably sitagliptin.

**15.** A process for preparing sitagliptin or retagliptin or evogliptin, preferably sitagliptin, comprising

(A) providing a compound of formula (II) obtained or obtainable from the method of any one of claims 1 to 12 or the compound of formula (II) according to claim 13;
(B) optionally, if $R^1$ is not hydrogen, deprotecting the compound of formula (II) thereby obtaining a compound of formula (II) wherein $R^1$ is hydrogen;
(C) reacting the compound of formula (II) of (A) or (B) with a compound of formula (V), (VI) or (VII)

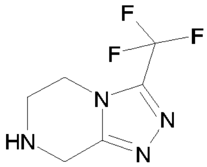

(V)

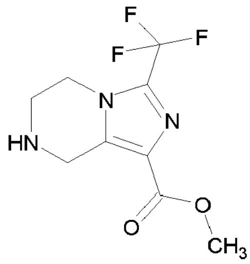

(VI)

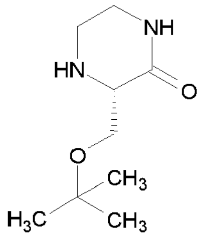

(VII)

thereby forming sitagliptin or retagliptin or evogliptin.

Europäisches Patentamt
European Patent Office
Office européen des brevets

# EUROPEAN SEARCH REPORT

Application Number
EP 22 17 2031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BUSS OLIVER ET AL: "[beta]-Phenylalanine Ester Synthesis from Stable [beta]-Keto Ester Substrate Using Engineered [omega]-Transaminases", MOLECULES, vol. 23, no. 5, 18 May 2018 (2018-05-18), page 1211, XP055975693, DOI: 10.3390/molecules23051211 * scheme 1; page 2, paragraph 4; table 1 * | 1-12 | INV. C12P7/40 |
| Y | CERIOLI ET AL: "Characterization of a novel amine transaminase from Halomonas elongata", JOURNAL OF MOLECULAR CATALYSIS B: ENZYMATIC,, vol. 120, 1 January 2015 (2015-01-01), pages 141-150, XP002774448, DOI: 10.1016/J.MOLCATB.2015.07.009 * figures 2,3; tables 3,4 * | 12 | |
| | | | TECHNICAL FIELDS SEARCHED (IPC) |
| X | KHOBRAGADE TARESH P. ET AL: "Synthesis of Sitagliptin Intermediate by a Multi-Enzymatic Cascade System Using Lipase and Transaminase With Benzylamine as an Amino Donor", FRONTIERS IN BIOENGINEERING AND BIOTECHNOLOGY, vol. 9, 6 October 2021 (2021-10-06), XP055975263, DOI: 10.3389/fbioe.2021.757062 | 1-9 | C12P |
| Y | * abstract; figure 1; table 1 * | 12 | |
| A,D | WO 2019/007146 A1 (UNIV ZHEJIANG TECHNOLOGY [CN] ET AL.) 10 January 2019 (2019-01-10) * [0031]; figures 1,3,6 * | 1-12 | |
| | -/-- | | |

~~The present search report has been drawn up for all claims~~

| Place of search | Date of completion of the search | Examiner |
|---|---|---|
| The Hague | 1 November 2022 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2
EPO FORM 1503 03.82 (P04C01)

Europäisches Patentamt
European Patent Office
Office européen des brevets

# EUROPEAN SEARCH REPORT

Application Number
EP 22 17 2031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | KOLLIPARA MANIDEEP ET AL: "Characterization of proteins from the 3N5M family reveals an operationally stable amine transaminase", APPLIED MICROBIOLOGY AND BIOTECHNOLOGY, vol. 106, no. 17, 6 August 2022 (2022-08-06), pages 5563-5574, XP055976254, Berlin/Heidelberg ISSN: 0175-7598, DOI: 10.1007/s00253-022-12071-1 Retrieved from the Internet: URL:https://link.springer.com/article/10.1007/s00253-022-12071-1/fulltext.html> ----- | | |
| | | | TECHNICAL FIELDS SEARCHED (IPC) |

~~The present search report has been drawn up for all claims~~

2

| Place of search | Date of completion of the search | Examiner |
|---|---|---|
| The Hague | 1 November 2022 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

Application Number
EP 22 17 2031

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-12

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 22 17 2031

The Search Division considers that the present European patent application does not comply with the requirements of unity of invention and relates to several inventions or groups of inventions, namely:

**1. claims: 1-12**

**A method for preparing a compound of formula (II) wherein R1 is selected from the group consisting of hydrogen atom, C1 to C10 alkyl, C6 to C12 aryl, C1 to C5 alkyl-C6 to C12 aryl, C3 to C10 heteroaryl, and C1 to C5R2 ,R3 ,R4 ,alkyl-C3 to C10 heteroaryl; R5 , R6 are independently of each other either a hydrogen atom or a halogen atom; R7 is a hydrogen atom or a protective group; the method comprising:**
**(a) Providing a compound of formula (I) wherein R1 is selected from the group consisting of hydrogen atom, C1 to C10 alkyl, C6 to C12 aryl, C1 to C5 alkyl-C6 to C12 aryl, C3 to C10 heteroaryl, and C1 to C5 alkyl-C3 to C10 heteroaryl; R2 , R3 , R4 , R5 and R6 have the same meaning as in the compound of formula (II);**
**(b) Providing an amine donor comprising an -NH2 group;**
**(c) Reacting the compound of formula (I) of (a) with the amine donor of (b) in the presence of an amine transaminase, wherein the amine transaminase comprises a polypeptide having at least 80 % sequence identity with the polypeptide of SEQ ID NO:1; thereby obtaining a reaction mixture comprising a compound of formula (II).**

---

**2. claim: 13**

**A compound of formula (II) obtained or obtainable from the aforementioned method.**

---

**3. claim: 14**

**Use of the compound of formula (II) obtained or obtainable from the method of any one of claims 1 to 12 or the compound of formula (II) according to claim 13 for the preparation of sitagliptin, retagliptin or evogliptin, preferably sitagliptin.**

---

**4. claim: 15**

**A process for preparing sitagliptin or retagliptin or evogliptin, preferably sitagliptin, comprising**
**(A) providing a compound of formula (II) obtained or obtainable from the method of any one of claims 1 to 12 or the compound of formula (II) according to claim 13;**
**(B) optionally, if R1 is not hydrogen, deprotecting the compound of formula (II) thereby obtaining a compound of formula (II) wherein R1 is hydrogen;**
**(C) reacting the compound of formula (II) of (A) or (B) with a compound of formula (V), (VI) or (VII) thereby forming**

Europäisches Patentamt
European Patent Office
Office européen des brevets

# LACK OF UNITY OF INVENTION SHEET B

Application Number
EP 22 17 2031

The Search Division considers that the present European patent application does not comply with the requirements of unity of invention and relates to several inventions or groups of inventions, namely:

**sitagliptin or retagliptin or evogliptin.**

---

## ANNEX TO THE EUROPEAN SEARCH REPORT ON EUROPEAN PATENT APPLICATION NO. EP 22 17 2031

This annex lists the patent family members relating to the patent documents cited in the above-mentioned European search report. The members are as contained in the European Patent Office EDP file on
The European Patent Office is in no way liable for these particulars which are merely given for the purpose of information.

01-11-2022

| Patent document cited in search report | Publication date | Patent family member(s) | Publication date |
|---|---|---|---|
| WO 2019007146 A1 | 10-01-2019 | CN 107384887 A | 24-11-2017 |
| | | WO 2019007146 A1 | 10-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 1391CHE2014 A
- CN 104805069 B
- CN 106801043 A
- CN 106191148 A
- WO 2019128894 A1
- WO 2019007146 A1
- CN 105331651 A
- IN 1391CHE2014


### Non-patent literature cited in the description


- **SAVILE et al.** *Science,* 2010, vol. 329, 305
- **HOU et al.** *Tetrahedron,* 2016, vol. 72, 4660
- **KIM et al.** *Enzyme Microb. Technol.,* 2019, vol. 120, 52
- **BUSTO et al.** *Adv. Synth. Catal.,* 2014, vol. 356, 1937
- *CHEMICAL ABSTRACTS,* 486460-21-3
- *CHEMICAL ABSTRACTS,* 1358715-37-3
- *CHEMICAL ABSTRACTS,* 1222102-49-9
- **SAVILE et al.** *Science,* 2010, vol. 329, 30